# EUROPÄISCHE PATENTSCHRIFT

(11) **EP 0 907 345 B1**
(45) Veröffentlichungstag und Bekanntmachung des Hinweises auf die Patenterteilung: **21.05.2003**
(21) Anmeldenummer: 97953717.2
(22) Anmeldetag: 29.11.1997
(51) Int. Cl.: A61K 7/00, A61K 7/48

(54) **ZWEIPHASEN-HAUTPFLEGEMITTEL**
DUAL PHASE SKIN CARE COMPOSITIONS
COMPOSITIONS A DEUX PHASES POUR LE SOIN DE LA PEAU

(30) Priorität: 07.12.1996 DE 19650952
(43) Veröffentlichungstag der Anmeldung: 14.04.1999
(73) Patentinhaber: Henkel Kommanditgesellschaft auf Aktien, 40589 Düsseldorf-Holthausen (DE)
(72) Erfinder: HEIDE, Barbara, D-47809 Krefeld (DE); BORDAT, Pascal, D-40699 Erkrath (DE)
(86) Internationale Anmeldenummer: EP9706665
(87) Internationale Veröffentlichungsnummer: WO98024399

(56) Entgegenhaltungen:
- DE-A- 3 841 775
- US-A- 4 335 103
- US-A- 5 059 414

## Beschreibung

Die Erfindung betrifft ein Zweiphasen-Hautpflegemittel, bevorzugt eine Zweiphasen-Hautcreme, die in einer Tube abgefüllt und aus dieser in Form paralleler, konzentrischer oder gestreifter Stränge auspreßbar ist.

Es sind verschiedene Tuben zur Abfüllung von Cremes bekannt, die aus zwei Komponenten bestehen und die vor dem Gebrauch getrennt gehalten werden sollen, oder die unterschiedlich gefärbt oder pigmentiert sind, und die aus der Tube in Form paralleler, konzentrischer oder gestreifter Stränge auspreßbar sind. Man hat aber diese Technik bisher nur bei Zahncremes in größerem Umfang realisiert.

Dies- mag daran liegen, daß in der Praxis zahlreiche Probleme auftreten, die im wesentlichen darin begründet sind, daß sich die Komponenten vor dem Auspressen aus der Tube nicht miteinander vermischen sollen, damit die getrennt gehaltenen Inhaltsstoffe dieser Komponenten nicht vorzeitig miteinander reagieren oder damit die unterschiedlichen Komponenten beim Auspressen aus der Tube noch als separate Phasen, z.B: in Form unterschiedlich gefärbter Streifen erkennbar bleiben.

So hat man z.B. gemäß DE-PS 820 268 vorgeschlagen, zwei Einzeltuben in konzentrischer Anordnung zu einer einheitlichen Tube zu vereinigen und die Öffnungen der beiden Einzeltuben so auszubilden, daß beim Druck auf die äußere Tube die beiden Komponenten als ein Strang mit einem Kern aus dem Inhalt der inneren Tube austreten.

Eine solche Anordnung ist aber teuer und großtechnisch schwer zu realisieren. In Streifen-Dispensem gemäß US.2,789,731, US 2,935,231, DE-A-2,141.436 besteht aber die Gefahr, daß die Trägercreme und die Streifencreme sich an der Phasengrenze mischen und in dem aus der Tube gedrückten Strang keine scharfen Phasengrenzen mehr erkennbar sind.

US-A-4 335 103 offenbart ein Hautpflegemittel, welches zwei getrennte, jeweils ein Verdickungsmittel enthaltende, plastisch fließende Komponenten umfaßt, die in einem Behälter abgefüllt sind, wobei es sich bei der ersten Komponente um ein Hydrogel und bei der zweiten Phase um eine Emulsion handelt. Die Phasen bleiben nebeneinander stabil, ohne sich zu vermischen.

US-A-5 059 414 offenbart Haar- und Hautpflegeprodukte, die zwei hochviskose, separate Komponenten enthalten. Die Komponenten, die in einem Pumpdispenser oder in einer Tube abgefüllt sind, bleiben nebeneinander stabil und lassen sich beispielsweise in Form gestreifter Stränge auspressen.

Es bestand daher die Aufgabe, für Haut- und Körperpflegemittel weitere Formulierungen zu finden, die eine Applikation von pasten- oder cremeförmigen Zubereitungen aus Tuben in Form von beim Ausdrücken mehrphasigen Strängen, deren Phasen bevorzugt unterschiedlich eingefärbt sind, möglich machen. Solche Produkte weisen nicht nur für den Anwender eine besondere Attraktivität auf, sondern ermöglichen es auch, Wirkstoffe auf die Haut zu bringen, die bei längerer Lagerung miteinander reagieren oder desaktiviert werden können.

Diese Aufgabe wurde erfindungsgemäß gelöst durch ein Haut- oder Körperpflegemittel, bestehend aus zwei separaten, plastisch fließenden Komponenten, die in einer Tube abgefüllt und aus dieser gemeinsam in Form paralleler, konzentrischer oder gestreifter Stränge beider Komponenten auspreßbar sind, wobei die erste Komponente eine kontinuierliche wäßrige Phase und die andere eine kontinuierliche ölige Phase aufweist.

Als Haut- oder Körperpflegemittel sind dabei solche Zubereitungen zu verstehen, die dem Schutz und der Pflege der Haut oder der Haare dienen und in Form von plastisch fließenden Zubereitungen auf der Hautoberfläche oder auf dem Haar verteilt werden und dort verbleiben. Als plastisch fließend wird ein rheologisches Verhalten bezeichnet, das für pasten-, creme- und gelförmige Systeme typisch ist und durch eine Fließgrenze gekennzeichnet ist, die das Produkt bei einer Schubspannung, die kleiner als diese Fließgrenze ist, wie einen Festkörper erscheinen läßt. Ein Fließen der Substanz wird erst beobachtet, wenn die Schubspannung größer als die Fließgrenze ist. Das rheologische Verhalten oberhalb der Fließgrenze kann mit Hilfe semi-empirischer Zustandsgleichungen wie beispielsweise der Bingham-Gleichung oder der Casson-Gleichung beschrieben werden.

Tuben, die geeignet sind, zwei separate, plastisch fließende Komponenten in Form konzentrischer oder gestreifter Produktstränge auszupressen, sind mehrfach beschrieben und für Zahnpasten mit farbigen Streifen seit langem im Handel. Diese Tuben sind so gestaltet, daß von der Tubenöffnung aus ein kleines Röhrchen in die Tube hineinragt, das an dem der Tubenöffnung zugewandten Ende Öffnungen aufweist. Die Tube wird in dem Raum um dieses Röhrchen herum mit einer z.B. gefärbten Zweitkomponente und darunter mit der Trägerkomponente befüllt. Das Röhrchen muß dabei in die Trägerkomponente hineinragen. Durch Druck auf die Tube wird die Trägerkomponente durch das Röhrchen und gegen die Zweitkomponente gedrückt, die ihrerseits durch die Öffnungen an den Strang der Trägercreme herangeführt und mit diesem herausgedrückt wird. Auf diese Weise werden beide Komponenten gemeinsam in Form eines Stranges ausgetragen. Je nach Größe und Form der Öffnungen kann der Strang schmale oder breitere Streifen der Zweitkomponente aufweisen. Wenn die Öffnung die Form eines Ringspalts aufweist, kann die Trägerphase von der Zweitphase ganz umhüllt sein. Durch die Gestaltung der Tubenöffnung kann der Produktstrang entweder zylindrisch, d.h. mit kreisrundem Querschnitt oder mit polygonem Querschnitt gebildet werden.

Als Komponente mit kontinuierlicher wäßriger Phase kann erfindungsgemäß ein wäßriges Gel, eine wäßrige Dispersion, eine Öl-in-Wasser-Emulsion oder eine Wasser-in-Öl-in-Wasser-Emulsion, eine wäßrige Mikroemulsion oder ein Gemisch solcher Systeme verwendet werden. Wichtig ist lediglich, daß Wasser die äußere, kontinuierliche Phase darstellt, daß also das System mit Wasser spontan verdünnbar ist.

Als wäßriges Gel kann dabei z.B. ein mit Hydrocolloiden oder Tensiden oder beiden verdicktes wäßriges System verwendet werden. Auch mit anorganischen Verdickungsmitteln, z.B. mit Kieselsäuren oder Schichtsilikaten verdickte wäßrige Systeme sind geeignet. Als wäßrige Dispersion ist eine Dispersion fester Partikel in einem wäßrigen Medium zu verstehen, z.B. eine Dispersion von Pigmenten, Wachsen oder Polymerpartikeln. Öl-in-Wasser-Emulsionen und Wasser-in-Öl-in-Wasser-Emulsionen sind die bekanntesten Grundlagen kosmetischer Hautpflegemittel. All diese genannten Komponenten mit kontinuierlicher wäßriger Phase sollten aber entweder durch geeignete Verdickungsmittel oder durch den Gehalt an dispergierter oder emulgierter Phase eine Viskosität von mehr als 0,1 Pa ^{·} s (20°C), bevorzugt von mehr als 1 Pa ^{·} s (20°C) (dynamische Viskosität oberhalb der Fließgrenze), aufweisen.

Als Komponente mit einer kontinuierlichen Ölphase eignen sich Öle und Fette, die entweder aufgrund ihrer Molekularstruktur oder durch Verdickung mit bekannten Verdickungsmitteln eine Viskosität von wenigstens 0,1 Pa ^{·} s, bevorzugt von wenigstens 1 Pa ^{·} s (20°C), aufweisen. Als solche eignen sich z.B. Vaseline (Petrolatum) oder andere bei 20°C salbenartige bzw. plastisch fließende pflanzliche, tierische oder synthetische Fette und Silikone. Öle mit niedrigerer Viskosität oder solche, die keine Fließgrenze aufweisen, sollten durch bekannte öllösliche Verdickungsmittel, z.B. durch Seifen, öllösliche Polymere, organisch modifizierte Schichtsilikate oder gelöste Wachse in einen plastisch fließenden Zustand überführt werden. Eine weitere Möglichkeit, Öle in einen plastisch fließenden Zustand zu überführen, ist die Einemulgierung einer diskontinuierlichen wäßrigen Phase, die ihrerseits gegebenenfalls eine Öl-in-Wasser-Emulsion sein kann. Auf diese Weise gebildeten Wasser-in-Öl-Emulsionen oder Öl-in-Wasser-in-Öl-Emulsionen können entweder durch die Viskosität der äußeren Ölphase oder durch die Menge der inneren wäßrigen Phase als plastisch fließende Systeme (Creme) erhalten werden.

Bevorzugt stellt die kontinuierliche wäßrige Phase ein wäßriges Gel oder eine Öl-in-Wasser-Emulsion und die kontinuieriche Ölphase ein verdicktes Öl oder eine Wasser-in-Öl-Emulsion dar. Die beiden plastisch fließenden Komponenten weisen bevorzugt eine Fließgrenze auf, die im Bereich von 50 bis 500 Pa (Pascal) bei 20°C liegt.

Für eine hohe Stabilität der Phasengrenze gegen ein Ausbluten oder eine Vermischung ist es auch vorteilhaft, wenn der Unterschied zwischen den Fließgrenzen der beiden Komponenten nicht größer als 20 %, bezogen auf die höhere Fließgrenze, ist.

Außer den Verdickungsmitteln oder der emulgierten Phase können die beiden Komponenten der erfindungsgemäßen Zweiphasen-Hautpflegemittel alle für den gewünschten Anwendungszweck üblichen Komponenten in den dafür geeigneten Konzentrationen enthalten.

Bevorzugt sind solche erfindungsgemäßen Haut- und Körperpflegemittel, bei denen die beiden Phasen unterschiedlich gefärbt oder pigmentiert sind, und auf diese Weise durch das farbliche Erscheinungsbild des aus der Tube tretenden Produktstrangs eine ästhetisch besonders attraktives Aussehen aufweisen.

Als Farbstoffe eignen sich dabei alle wasserlöslichen bzw. öllöslichen Farbstoffe, die für die Anfärbung kosmetischer Produkte zugelassen sind. So kann man z.B. die Ölphase mit öllöslichen Farbstoffen einfärben oder die wäßrige Phase mit dispergierten Pigmenten eintrüben. Man kann auch die wäßrige Phase als klares, eingefärbtes Gel und die Ölphase als weiße, gegebenenfalls pigmentierte Wasser-in-Öl-Creme ausführen. Eine weitere Spielart besteht darin, daß man z.B. die ölige Phase als klares, gegebenenfalls gefärbtes Gel und die wäßrige Phase als pigmentierte, weiße Öl-in-Wasser-Creme formuliert.

Im Zusammenspiel mit der Gestaltung der Tubenöffnung und mit der Zahl, Größe und Form der Öffnungen, durch welche die Zweitkomponente aus der Tube austritt. kann eine ästhetisch sehr vielseitige Gestaltung des Produktstrangs erreicht werden.

Darüber hinaus können den beiden Komponenten unterschiedliche kosmetische Wirkstoffe zugesetzt werden, so daß z.B. die wäßrige Phase reinigende und die ölige Phase pflegende Wirkstoffe enthalten kann. Auch kann man der wäßrigen Phase z.B. Liposomen zusetzen, die in der öligen Phase instabil wären. Der öligen Phase könnte man z.B. Wirkstoffe zusetzen, die in Gegenwart von Wasser instabil oder unlöslich sind.

Das folgende Beispiel soll den Patentgegenstand näher erläutern:

### Beispiel

In einen Tubenspender gemäß DE-A-3 841 775 wurde eine O/W-Trägercreme und eine W/O-Streifencreme der folgenden Rezepturen eingefüllt. Dabei bildete die W/O-Nachtcreme die Streifencreme und die O/W-Tagescreme die Trägercreme. Insgeamt wurden in eine 125 ml-Tube 118 ml Trägercreme und 7 ml Streifencreme eingefüllt.

| **Trägercreme (O/W)** | |
|---|---|
| Stearinsäure | 8,0 Gew.-% |
| Cetyl-/Stearylalkohol | 1,5 Gew.-% |
| Cetyl-/Sterylalkohol + 20 Mol EO | 2,0 Gew.-% |
| 2-Octyldodecanol | 5,0 Gew.-% |
| Cera Alba | 3,0 Gew.-% |
| Paraffinum Liquidum | 10,0 Gew.-% |
| p-Hydroxybenzoesäure-propylester | 0,3 Gew.-% |
| Tocopherylacetat | 2,0 Gew.-% |
| Benzophenone-3 | 1,0 Gew.-% |
| Propylenglycol | 5,0 Gew.-% |
| Glycerin | 5,0 Gew.-% |
| p-Hydroxybenzoesäure-methylester | 0,3 Gew.-% |
| Triethanolamin | 0,3 Gew.-% |
| Wasser | ad 100 Gew.-% |

| **Streifencreme (W/O)** | |
|---|---|
| Dehymuls F | 8,0 Gew.-% |
| Sheabutter | 5,0 Gew.-% |
| Petrolatum | 10,0 Gew.-% |
| Paraffinum liquidum | 5,0 Gew.-% |
| Methylparaben | 0,3 Gew.-% |
| Tocopherylacetat | 2,0 Gew.-% |
| Propylenglycol | 5,0 Gew.-% |
| Glycerin | 5,0 Gew.-% |
| Magnesiumsulfat | 0,5 Gew.-% |
| Phenoxyethanol | 1,0 Gew.-% |
| Acid Orange 24 (C.I. 20170) | 0,5 Gew.-% |
| Wasser | ad 100 Gew.-% |

Beim Ausdrücken der Tube wurde ein quadratischer Strang der Trägercreme mit orangefarbenen Streifen der W/O-Creme an den Kanten des Trägerstranges erhalten. Die Phasengrenze zwischen der Trägercreme und den orangefarbenen Streifen blieb über 4 Wochen stabil.

## Patentansprüche

1. Haut- und Körperpflegemittel, bestehend aus zwei getrennten, plastisch fließenden Komponenten, die in einer Tube abgefüllt und aus dieser gemeinsam in Form paralleler, konzentrischer oder gestreifter Stränge beider Komponenten auspreßbar sind, **dadurch gekennzeichnet, daß** die erste Komponente eine kontinuierliche wäßrige Phase und die zweite Komponente eine kontinuierliche Ölphase aufweist.

2. Haut- oder Körperpflegemittel nach Anspruch 1, **dadurch gekennzeichnet, daß** die beiden Phasen unterschiedlich gefärbt oder pigmentiert sind.

3. Haut- oder Körperpflegemittel nach Anspruch 1 oder 2, **dadurch gekennzeichnet, daß** die kontinuierliche wäßrige Phase ein wäßriges Gel oder eine Öl-in-Wasser-Emulsion und die kontinuierliche Ölphase ein verdicktes Öl oder eine Wasser-in-Öl-Emulsion ist.

4. Haut- oder Körperpflegemittel nach einem der Ansprüche 1 bis 3, **dadurch gekennzeichnet, daß** die wäßrige und die ölige Phase eine Fließgrenze von 50 bis 500 Pa bei 20°C aufweisen.

## Claims

1. A skin-care and body-care formulation consisting of two separate, plastically flowing components accommodated in a tube from which they can be extruded together in the form of parallel, concentric or striped strands of both components, **characterized in that** the first component comprises a continuous aqueous phase and the second component a continuous oily phase.

2. A skin-care and body-care formulation as claimed in claim 1, **characterized in that** the two phases are differently colored or pigmented.

3. A skin-care and body-care formulation as claimed in claim 1 or 2, **characterized in that** the continuous aqueous phase is an aqueous gel or an oil-in-water emulsion and the continuous phase is a thickened oil or a water-in-oil emulsion.

4. A skin-care and body-care formulation as claimed in any of claims 1 to 3, **characterized in that** the aqueous phase and the oily phase have yield points of 50 to 500 Pa at 20°C.

## Revendications

1. Produit de soins pour la peau et pour le corps, consistant en deux composants séparés, qui s'écoulent d'une manière plastique, qui sont conditionnés en un tube, et qui peuvent être pressés de celui-ci conjointement en deux filaments parallèles, concentriques ou en bandes, de ces deux composants,
**caractérisé en ce que**
le premier composant possède une phase aqueuse continue et le deuxième composant possède une phase huileuse continue.

2. Produit de soins pour la peau et pour le corps selon la revendication 1,
**caractérisé en ce que**
les deux phases sont différemment colorées ou pigmentées.

3. Produit de soins pour la peau et pour le corps selon l'une quelconque des revendications 1 ou 2,
**caractérisé en ce que**
la phase aqueuse continue est un gel aqueux ou une émulsion huile dans l'eau et la phase huileuse continue est une huile épaissie ou une émulsion eau dans l'huile.

4. Produit de soins pour la peau et pour le corps selon l'une quelconque des revendications 1 à 3,
**caractérisé en ce que**
la phase aqueuse et la phase huileuse possèdent une limite d'écoulement allant de 50 à 500 Pa à 20°C.
